# EUROPÄISCHE PATENTSCHRIFT

(11) **EP 1 203 594 B1**
(45) Veröffentlichungstag und Bekanntmachung des Hinweises auf die Patenterteilung: **30.11.2005**
(21) Anmeldenummer: 01125400.0
(22) Anmeldetag: 31.10.2001
(51) Int. Cl.: A61M 16/04

(54) **Tracheostomiedilatator**
Tracheostomy dilator
Dilatateur pour Trachéotomie

(30) Priorität: 03.11.2000 DE 10054527; 11.09.2001 DE 10144534
(43) Veröffentlichungstag der Anmeldung: 08.05.2002
(73) Patentinhaber: Willy Rüsch GmbH, 71394 Kernen-Rommelshausen (DE)
(72) Erfinder: Frova, Giulio, Prof. Dr. Med., 20121 Mailand (IT); Quintel Michael, Prof. Dr. med., 68167 Mannheim (DE); Mailänder, Werner, Dipl.-Ing., 71404 Korb (DE); Kaczorowski, Heiko, Dr. rer. nat., 70188 Stuttgart (DE); Ranzinger, Gisbert, Dr. med., 73773 Aichwald (DE)
(74) Vertreter: Kohler Schmid Möbus

(56) Entgegenhaltungen:
- EP-A- 0 530 595
- WO-A-91/07202
- WO-A-99/44665
- DE-A- 1 541 237
- GB-A- 2 313 316
- US-A- 4 026 296

## Beschreibung

Derartige Tracheostomiedilatatoren sind im Zusammenhang mit der Punktionstracheostomie nach Ciaglia et al., "Elective Percutaneous Dilatational Tracheostomy" Chest 1985, Heft 6, S. 715-719, bekannt geworden. Bei der bekannten Punktionstracheostomie wird nach bronchoskopisch kontrollierter, transcutaner Punktion der Trachea unterhalb des Ringknorpels, vorzugsweise zwischen dem zweiten und dritten Trachealring, über einen Seldinger-Draht mit abgestuften Dilatatoren die Trachea sukzessive aufgedehnt, und nach gewünschter Aufdehnung wird in die Trachea eine Tracheostomiekanüle eingebracht. Diese Tracheostomiemethode hat sich mittlerweile zumindest für ein streng selektiertes Krankengut durchgesetzt. Die bekannte Methode birgt jedoch auch Risiken für den Patienten, so dass nur sehr erfahrene Anästhesisten diese Methode anwenden sollten. Das bekannte Verfahren ist zeitraubend, und bei jedem Bougierungsvorgang besteht die Gefahr der Verletzung der trachealen Hinterwand.

Eine weiterentwickelte bekannte Methode der percutanen Dilatationstracheostomie, die aus Wolfgarten et al. "Punktionstracheotomie mit einmaliger Trachealdilatation", Chirurg 2000, S. 63-65 u. 723-724 bekannt geworden ist, besteht beispielsweise darin, dass zwischen Ringknorpel und Incisura jugularis etwa in Höhe des dritten Trachelrings eine Hautincision vorgenommen wird. Danach wird das Hautgewebe und die Halsmuskulatur mit einer Klemme so weit gespreizt, bis die Trachea sichtbar wird. Mit einer Hohlnadel wird unter bronchoskopischer Kontrolle die Trachea durch die Hautincision punktiert, ein Führungsdraht unter Sichtkontrolle in die Trachea vorgeschoben, und über den Führungsdraht erfolgt eine einmalige Bougierung mittels eines konischen Dilatators auf eine Größe, die für die einzubringende Tracheostomiekanüle notwendig ist.

Den bekannten Dilatationsverfahren ist gemein, dass mit einer großen Vorschubkraft und einem langen Vorschubweg der Stomakanal und die Trachealvorderwand geweitet werden muss. Dabei kann die Spitze des Dilatators bedrohlich nahe an die Trachealhinterwand gelangen und dort Verletzungen erzeugen.

Deshalb erscheint die Einmalbougierung noch weniger kontrollierbar zu sein als die mehrstufige Bougierung nach Ciaglia et al., weil die Vorschubkräfte bei der Einmalbougierung größer sind und sich der Vorschubweg über eine mehrstufige Bougierung besser kontrollieren lässt.

Aus der WO91/07202 ist eine Vorrichtung zur Durchführung einer perkutanen Tracheostomie bekannt geworden, die einen rohrförmigen Penetrator mit geringem Durchmesser aufweist, der ein abgeschrägtes Ende hat, einen Atemluftkanal mit größerem Durchmesser, einen Gewebeaufweiter und eine Postionierungseinrichtung umfasst. Das freie Ende durchdringt das Gewebe des Patienten, um eine kleine Punktierung zu erreichen, die der Gewebeaufweiter aufweitet, so dass der Atemluftkanal hindurchpasst. Der Gewebeaufweiter ist als Gewinde ausgebildet. Dokument WO91/07202 ist als nächstliegen der Stand der Technik angesehen und offenbart einen Tracheostomiedilatator gemäß dem Oberbegriff des Anspruchs 1.

Aus der EP 0 530 595 A1 ist eine Trokarhülse bekannt, die in der Lage ist, starre, gekrümmte Hilfsinstrumente aufzunehmen. Die Trokarhülse ist zumindest auf einem Teil ihrer Länge biegsam ausgebildet, so dass sich ihre Form durch elastisches Biegen, im Übrigen aber knickfest, den Krümmungen der Hilfsinstrumente anzupassen vermag.

Aus der DE 1 541 237 ist ein Trokar zur Herstellung der Verbindung des Innenraums eines Hohlorgans eines Tieres mit der Atmosphäre über eine durch den Trokar hindurchgehende, vorzugsweise zentral angeordnete Bohrung, bekannt geworden, wobei der Trokar einen gegen die Außenfläche der Bauchwand des Rindes sich anlegenden Anschlag aufweist, der auf seiner unteren Seite in einen vorzugsweise zylinderförmigen Schaft übergeht, wobei eine Spiralschraube vorgesehen ist, die sich an den Schaft anschließt und deren oberster Spiralgang als Anschlag ausgebildet ist, der sich gegen die Innenwand des Hohlorgans anlegt.

Aus der GB 2 313 316 A ist ein Thoraxdrainageinstrument mit einem Gewinde bekannt geworden. Das Gewinde dient dazu, Muskelfasern zu trennen, um eine Durchgangsöffnung zu schaffen.

Aufgabe der vorliegenden Erfindung ist es, die bekannten Tracheostomiedilatatoren derart weiterzubilden, dass das Gewebe des Patienten besonders schonend aufgeweitet werden kann.

Diese Aufgabe wird erfindungsgemäß durch einen Tracheostomiedilatator mit den Merkmalen des Anspruchs 1 gelöst.

Der erfindungsgemäße Tracheostomiedilatator hat damit den wesentlichen Vorteil, dass beim Dilatationsvorgang, auch bei einer einmaligen Dilatation, die Kompression auf die Trachealvorderwand auf ein Minimum reduziert wird. Der erfindungsgemäße Dilatator wird auf einen in bekannter Weise in die Trachea eingeführten Führungsdraht aufgefädelt und danach in das Gewebe eingeschraubt, bis er in gewünschter Weise in die Trachea vorgedrungen ist. Der erfindungsgemäße Dilatator benötigt keinen vom Anwender aufgebrachten Vorschubimpuls in transversaler Richtung, weil über die Gewindeausbildung ein Vorschub des Dilatators automatisch, entsprechend der gewählten Gewindesteigung erfolgt.

Mit dem erfindungsgemäßen Dilatator wird die Trachealvorderwand sehr schonend durchdrungen und nicht wie bei einer herkömmlichen Dilatation, die im Wesentlichen mit hohen Vorschubkräften einhergeht, die Vorderwand ins Innenlumen der Trachea protrudiert, so dass die Dilatatorspitze bei einer herkömmlichen Dilatation bedrohlich nahe an die Trachealhinterwand herankommen kann. Diese Gefahr ist bei der Verwendung des erfindungsgemäßen Dilatators nicht gegeben, weil die Dilatation in einem schraubenförmigen Bewegungsablauf erfolgt. Gewünscht ist nur die Dilatation; unerwünscht, weil gefahrenträchtig, ist die Kompression der Trachealwand. Bei einer Schraubdilatation wird die Kompression auf die Trachealvorderwand minimiert. Die Vorschubkräfte sind bei einer Schraubdilatation in einem hohen Maße über die Drehung des Dilatators kontrolliert steuerbar und über die gewählte Gewindesteigung fest vorgegeben. So kann eine gesicherte stetige Dilatation in radialer Richtung erfolgen, ohne dass ein zu starker Druck in axialer Richtung auf das zu dilatatierende Gewebe ausgeübt wird.

Bei der Erfindung verjüngt sich der Stab im Abschnittsbereich mit dem auf dem Stab ausgebildeten ersten Gewinde zum freien patientenseitigen Ende des Stabes hin. Dies hat den Vorteil, dass das zu weitende Gewebe schonend gedehnt wird. Je nach dem wie stark dieser Abschnittsbereich konisch verjüngt ist, wird die notwendige Dilatation auf einem kürzeren oder längeren Wegabschnitt vorgenommen.

Weiterhin ist das erste Gewinde als selbstschneidendes Gewinde ausgebildet. Dies hat den Vorteil, dass der Dilatator mit geringem äußeren Kraftaufwand in das zu weitende Gewebe eindringen, d.h. eingedreht werden kann.

Ist das erste Gewinde des Tracheostomiedilatators über die Länge des ersten Gewindes mit unterschiedlich stark ansteigenden Gewindegängen ausgebildet, so ist es möglich, in einem ersten Dilatationsschritt über mehrere Dilatatordrehungen nur eine geringe Weitung des Gewebes zu erzeugen und in einem beispielsweise zweiten Dilatationsschritt, bei dem die Gewindespitze des erfindungsgemäßen Dilatators schon fest im Gewebe verankert ist, eine größere Dilatation bei einer geringfügigeren Drehbewegung, die mit einem größeren axialen Vorschub einhergeht, zu erreichen.

Ist das freie patientenseitige Ende des Stabes zusätzlich angeschrägt, so kann die Dilatatorspitze besonders schonend in das an die Spitze angrenzende Gewebe eindringen.

Bevorzugt ist der Durchmesser des Innenlumens an den Außendurchmesser eines Führungsdrahtes so angepasst, dass das Innenvolumen möglichst spaltfrei von dem Führungsdraht ausgefüllt ist. Dies hat den Vorteil, dass beim Dilatationsvorgang kein Gewebe in das Innenlumen eindringen kann.

Der Stab weist ein sich an das erste Gewinde anschließendes zweites Gewinde auf. Durch diese Maßnahme kann zum einen der weitere Verschub des Dilatators kontrolliert erfolgen. Zum anderen wird der Dilatator durch das zweite Gewinde lagefixiert. Das zweite Gewinde kann bis zum patientenabgewandten Ende, also entlang des gesamten zylindrischen Schaftes, ausgebildet sein oder nur im patientenseitigen Abschnitt des zylindrischen Schaftes. Die Gewindesteigung kann der des ersten Gewindes entsprechen oder davon abweichen. Auch ist es denkbar, dass sich die Gewindesteigung des zweiten Gewindes entlang des Schaftes verändert. Das zweite Gewinde kann einerseits als auf den Schaft aufgesetzter Steg oder andererseits als Vertiefung in der Stegwand ausgebildet sein.

Das erste und zweite Gewinde weisen eine Oberfläche auf, die in Berührung mit einem Gewebe reibungsarme Eigenschaften aufweist, wobei die Außenoberfläche eine hydrophile Beschichtung aufweist und/oder mit einem Gleitgel benetzt ist. So kann der Gewindeabschnitt des Dilatators mit geringem Kräfteaufwand in das zu dilatierende Gewebe kontrolliert eingedreht werden.

Um eine kontrollierte Drehbewegung des Dilatators auslösen zu können, ist am patientenabgewandten Ende des Stabes bevorzugt ein Griffkopf ausgebildet, damit der Anwender den erfindungsgemäßen Schraubdilatator kontrolliert drehen kann.

Ist der erfindungsgemäße Tracheostomiedilatator aus einem kochfesten Kunststoff oder aus einem Metall hergestellt, so kann er mehrfach sterilisiert werden und muss nicht zwingend als Einmalinstrument eingesetzt werden.

In weiterer bevorzugter Ausgestaltung der Erfindung kann der Tracheostomiedilatator aus zwei Halbschalen gefertigt sein, die entweder über ein Filmscharnier miteinander verbunden sind oder in einer anderen Art und Weise für den Dilatationsprozess miteinander verbunden sind. Ist der Dilatationsvorgang abgeschlossen, so kann nach plazierter Tracheostomiekanüle der Schraubdilatator zurückgezogen werden und im patientenfreien Bereich vom Führungsdraht abgeklappt werden. Diese Ausführungsform hat den Vorteil, dass der Schraubdilatator nicht über die gesamte Länge des Führungsdrahtes zurückgezogen werden muss.

In einer weiteren erfindungsgemäßen Ausführungsform kann auf der Außenoberfläche des erfindungsgemäßen Schraubdilatators schon eine Tracheostomiekanüle angeordnet sein. Dies hat den Vorteil, dass gleichzeitig mit dem Dilatationsvorgang die Tracheostomiekanüle in die Trachea vorgeschoben wird.

Mit dem erfindungsgemäßen Schraubdilatator können die nach wie vor vorhandenen Risiken bei einer percutanen Dilatationstracheostomie reduziert werden, weil die Dilatation über eine radiale Bewegungskomponente ausgelöst wird.

Weitere Vorteile ergeben sich aus der Beschreibung und der beigefügten Zeichnung. Bei den Darstellungen der Figuren 1 und 2 handelt es sich um Beispiele, die das Verständnis der Erfindung erleichtern. Die nachfolgend in Figur 3 beschriebene Aus-führungsform ist als beispielhafte Darstellung eines erfindungsgemäßen Tracheostomiedilatators zu verstehen. Die in den Figuren abgebildete Ausführungsform ist stark schematisiert dargestellt und ist nicht maßstäblich zu verstehen. Es zeigt:

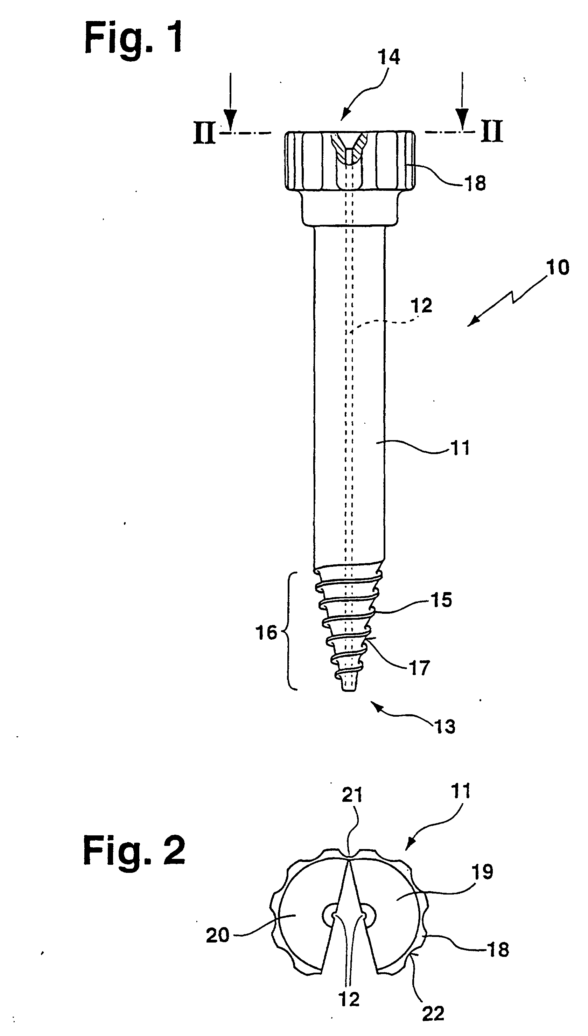

- Fig. 1: eine Seitenansicht eines Tracheostomiedilatators;

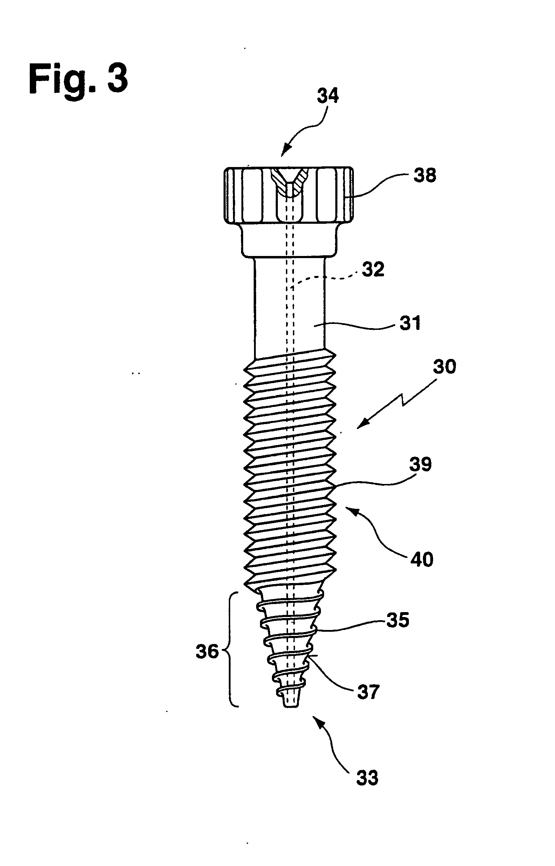

- Fig. 2: eine Draufsicht gemäß II-II von Fig. 1;
- Fig. 3: eine Seitenansicht eines erfindungsgemäßen Tracheostomiedilatators.

**Fig. 1** zeigt mit 10 einen Tracheostomiedilatator, der aus einem Stab 11 mit einem Innenlumen 12 gebildet ist. Der Stab 11 ist aus einem biegesteifen Material, wie einem kochfesten Kunststoff oder einem Metall hergestellt. Der Stab 11 kann auch gebogen sein. Das Innenlumen 12 durchläuft den Stab 11 in axialer Richtung und ist als Durchgangslumen ausgebildet, so dass durch den Stab 11 ein in der Fig. 1 nicht gezeigter Führungsdraht geschoben werden kann.

Am patientenseitigen Ende 13 ist der Tracheostomiedilatator 10 verjüngt, und in das freie Ende kann ein Führungsdraht eingeschoben werden, der am patientenabgewandten Ende 14 aus dem Tracheostomiedilatator 10 herausragt. Zusätzlich ist am patientenseitigen Ende 13 ein erstes Gewinde 15 ausgebildet, das sich über einen Abschnittsbereich 16 erstreckt. Das erste Gewinde 15 kann über die axiale Erstrekkung des ersten Gewindes 15 unterschiedliche Gewindesteigungen aufweisen und so ausgebildet sein, dass es selbstschneidende Eigenschaften aufweist. Die freie Spitze ist am patientenseitigen Ende 13 angeschrägt.

Eine Außenoberfläche 17 des ersten Gewindes 15 ist bevorzugt glatt ausgebildet, so dass kein Gewebe in den Gewindegängen anhaften kann. Die Außenoberfläche 17 kann zusätzlich noch eine hydrophile Beschichtung und/oder Gelbenetzung aufweisen.

Am patientenabgewandten Ende 34 ist ein Griffkopf 38 ausgebildet, damit ein Anwender den Tracheostomiedilatator 30 gut greifen und bei Bedarf kontrolliert drehen kann.

Fig. 2 zeigt den Tracheostomiedilatator 10 aus Fig. 1 in einer Draufsicht gemäß II-II von Fig. 1. Der Stab 11 ist aus einer ersten Halbschale 19 und aus einer zweiten Halbschale 20 zusammengesetzt, die über ein Filmscharnier 21 zusammengehalten werden. Werden die Halbschalen 19, 20 aufgeklappt, so teilt sich das Innenlumen 12 und ein im Innenlumen 12 geführter Führungsdraht kann aus dem Tracheostomiedilatator herausgenommen werden. Der Griffkopf 18 weist eine Kontur 22 derart auf, dass ein Anwender den Tracheostomiedilatator kontrolliert halten und drehen kann.

**Fig. 3** zeigt mit 30 einen Tracheostomiedilatator, der aus einem Stab 31 mit einem Innenlumen 32 gebildet ist. Der Stab 31 ist aus einem biegesteifen Material, wie einem kochfesten Kunststoff oder einem Metall hergestellt. Der Stab 31 kann in weiteren Ausführungsformen auch gebogen sein. Das Innenlumen 32 durchläuft den Stab 31 in axialer Richtung und ist als Durchgangslumen ausgebildet, so dass durch den Stab 31 ein in der Fig. 3 nicht gezeigter Führungsdraht geschoben werden kann.

Am patientenseitigen Ende 33 ist der Tracheostomiedilatator 30 verjüngt, und in das freie Ende kann ein Führungsdraht eingeschoben werden, der am patientenabgewandten Ende 34 aus dem Tracheostomiedilatator 30 herausragt. Zusätzlich ist am patientenseitigen Ende 33 ein erstes Gewinde 35 ausgebildet, das sich über einen Abschnittsbereich 36 erstreckt. Das erste Gewinde 35 kann über die axiale Erstrekkung des ersten Gewindes 35 unterschiedliche Gewindesteigungen aufweisen und ist so ausgebildet, dass es selbstschneidende Eigenschaften aufweist. Die freie Spitze ist am patientenseitigen Ende 33 angeschrägt. An das erste Gewinde 35 schließt sich ein zweites Gewinde 39 an, das am zylindrischen Schaftabschnitt 40 des Stabes 31 ausgebildet ist. Im Beispiel erstreckt sich das zweite Gewinde 39 nur über einen Teil des zylindrischen Schaftabschnitts 40.

Das erste Gewinde (35) und das zweite Gewinde (39) weisen eine Außenoberfläche (37) auf, die eine hydrophile Beschichtung aufweist und/oder mit einem Gleitgel benetzt ist, so dass Kein Gewebe in den Gewindegängen anhaften Kann.

## Patentansprüche

1. Tracheostomiedilatator (30), bestehend aus einem Stab (31) mit einem beiden Ends offenen Innenlumen (32), das sich vom patientenseitigen Ende (33) bis zum patientenabgewandten Ende des Stabes (31) erstreckt, wobei am patientenseitigen Ende (33) des Stabes (31) ein erstes Gewinde (35) ausgebildet ist, und sich der Stab (31) im Abschnittsbereich (36) mit dem auf dem Stab (31) ausgebildeten ersten Gewinde (35) zum patientenseitigen Ende (33) hin verjüngt, **dadurch gekennzeichnet, dass** das erste Gewinde (35) als selbstschneidendes Gewinde ausgebildet ist, wobei der Stab (31) ein sich an das erste Gewinde (35) anschließendes zweites Gewinde (39) aufweist, wobei das erste Gewinde (35) und das zweite Gewinde (39) eine Außenoberfläche (37) aufweist, die in Berührung mit einem Gewebe reibungsarme Eigenschaften aufweist, wobei die Außenoberfläche (37) eine hydrophile Beschichtung aufweist und/oder mit einem Gleitgel benetzt ist.

2. Tracheostomiedilatator (30) nach Anspruch 1, **dadurch gekennzeichnet, dass** das erste Gewinde (35) über seine Länge gesehen unterschiedliche Gewindesteigungen aufweist.

3. Tracheostomiedilatator (30) nach einem der Ansprüche 1 oder 2, **dadurch gekennzeichnet, dass** das freie patientenseitige Ende (33) des Stabes (31) abgeschrägt ist.

4. Tracheostomiedilatator (30) nach einem der Ansprüche 1 bis 3, **dadurch gekennzeichnet, dass** der Durchmesser des Innenlumens (32) an den Außendurchmesser eines Führungsdrahtes angepasst ist.

5. Tracheostomiedilatator (30) nach einem der Ansprüche 1 bis 4, **dadurch gekennzeichnet, dass** am patientenabgewandten Ende (34) des Stabes (31) ein Griffkopf (38) ausgebildet ist.

6. Tracheostomiedilatator (30) nach einem der Ansprüche 1 bis 5, **dadurch gekennzeichnet, dass** der Stab (31) aus einem kochfesten Kunststoff oder Metall hergestellt ist.

7. Tracheostomiedilatator (30) nach einem der Ansprüche 1 bis 6, **dadurch gekennzeichnet, dass** der Stab (31) in zwei Halbschalen teilbar oder aufklappbar ist.

8. Tracheostomiedilatator (30) nach einem der Ansprüche 1 bis 7, **dadurch gekennzeichnet, dass** auf der Außenoberfläche des Stabes (31) eine Tracheostomiekanüle angeordnet ist.

## Claims

1. Tracheostomy dilator (30) consisting of a rod (31) with an inner lumen (32) which is open at both ends and extends from the end (33) facing the patient to the end of the rod (31) facing away from the patient, wherein the end (33) of the rod (31) facing the patient is provided with a first thread (35) and the section (36) of the rod (31) which is provided with the first thread (35) tapers towards the end (33) facing the patient, **characterized in that** the first thread (35) is formed as automatically cutting thread, wherein the rod (31) comprises a second thread (39) connecting to the first thread (35), wherein the first thread (35) and the second thread (39) have an outer surface (37) which shows little friction when contacting tissue, wherein the outer surface (37) has a hydrophilic coating and/or is wetted with a lubricant.

2. Tracheostomy dilator (30) according to claim 1, **characterized in that** the pitch of the first thread (35) differs, as viewed over its length.

3. Tracheostomy dilator (30) according to any one of the claims 1 or 2, **characterized in that** the free end (33) of the rod (31) facing the patient is chamfered.

4. Tracheostomy dilator (30) according to any one of the claims 1 through 3, **characterized in that** the diameter of the inner lumen (32) is adjusted to the outer diameter of a guiding wire.

5. Tracheostomy dilator (30) according to any one of the claims 1 through 4, **characterized in that** the end (34) of the rod (31) facing away from the patient is provided with a gripping head (38).

6. Tracheostomy dilator (30) according to any one of the claims 1 through 5, **characterized in that** the rod (31) is produced from a boil proof plastic material or metal.

7. Tracheostomy dilator (30) according to any one of the claims 1 through 6, **characterized in that** the rod (31) can be divided or unfolded into two half shells.

8. Tracheostomy dilator (30) according to any one of the claims 1 through 7, **characterized in that** a tracheostomy cannula is disposed on the outer surface of the rod (31).

## Revendications

1. Dilatateur pour trachéotomie (30) formé par une tige (31) avec un conduit intérieur (32), qui est ouvert aux deux extrémités et s'étend depuis l'extrémité du côté patient (33) jusqu'à l'extrémité, opposée au patient, de la tige (31), un premier filetage (35) étant réalisé sur l'extrémité du côté patient (33) de la tige (31) et la tige (31) se rétrécissant vers l'extrémité du côté patient (33) dans la zone (36) munie du premier filetage (35) réalisé sur la tige (31), **caractérisé en ce que** le premier filetage (35) est un filetage autotaraudeur, la tige (31) comportant un deuxième filetage (39) dans le prolongement du premier filetage (35), le premier filetage (35) et le deuxième filetage (39) ayant une surface extérieure (37) avec des propriétés de faible frottement en cas de contact avec un tissu, la surface extérieure (37) étant revêtue d'une couche hydrophile et/ou étant humectée par un gel facilitant le glissement.

2. Dilatateur pour trachéotomie (30) selon la revendication 1, **caractérisé en ce que** le premier filetage (35) comporte différents pas de vis sur sa longueur.

3. Dilatateur pour trachéotomie (30) selon la revendication 1 ou 2, **caractérisé en ce que** l'extrémité libre (33), du côté patient, de la tige (31) est biseautée.

4. Dilatateur pour trachéotomie (30) selon l'une quelconque des revendications 1 à 3, **caractérisé en ce que** le diamètre du conduit intérieur (32) est ajusté au diamètre extérieur d'un fil de guidage.

5. Dilatateur pour trachéotomie (30) selon l'une quelconque des revendications 1 à 4, **caractérisé en ce qu'**une tête formant poignée (38) est réalisée sur l'extrémité (34), opposée au patient, de la tige (31).

6. Dilatateur pour trachéotomie (30) selon l'une quelconque des revendications 1 à 5, **caractérisé en ce que** la tige (31) est réalisée dans une matière plastique ou un métal résistant à l'ébullition.

7. Dilatateur pour trachéotomie (30) selon l'une quelconque des revendications 1 à 6, **caractérisé en ce que** la tige (31) peut être séparée ou être dépliée en deux demi-coques.

8. Dilatateur pour trachéotomie (30) selon l'une quelconque des revendications 1 à 7, **caractérisé en ce qu'**une canule de trachéotomie est agencée sur la surface extérieure de la tige (31).
